# EUROPEAN PATENT SPECIFICATION

(11) **EP 1 414 508 B1**
(45) Date of publication and mention of the grant of the patent: **20.04.2011**
(21) Application number: 02756297.4
(22) Date of filing: 26.06.2002
(51) Int. Cl.: A61M 11/00, A61M 15/00, A61M 16/10

(54) **AN IMPROVED AEROSOL ACTUATOR**
VERBESSERTER AEROSOL-REGLER
DISPOSITIF D'ACTIONNEMENT D'UN AEROSOL

(30) Priority: 26.06.2001 US 301296 P
(43) Date of publication of application: 06.05.2004
(60) Divisional application: 10010834.9
(73) Proprietor: NORTON HEALTHCARE LIMITED, Swiss Cottage London NW6 3RZ (GB)
(72) Inventor: LANGFORD, Alan, Herts EN 11 9DG (GB); HENNESSY, Fergal, Waterford City, County Waterford (IE); HOLROYD, Michael, Cambridge CB2 5JZ (GB)
(74) Representative: Cottam, David William
(86) International application number: PCT/US2002/020136
(87) International publication number: WO 2003/002169

(56) References cited:
- WO-A1-00/37336
- WO-A1-99/47195
- US-A- 3 598 294
- US-A- 5 213 266
- US-A- 5 894 964

## Description

The present invention relates to the field of inhalers used for the delivery of medicaments indicated for the treatment, or alleviation of the effects of any ailment including respiratory complaints and various systemic diseases, via the delivery of a medicament by the pulmonary route.

Current conventional actuators used for the delivery of medicaments include a sump, or well, located in a valve stem block adjacent to the actuator outlet. The shape of the sump is dictated primarily by engineering requirements such as the strength of small moulding pins. Current sumps have angles and corners where the medicament being delivered can accumulate and/or deposit, giving rise to a reduction in the dose available and an increase in the occurrence of the blockage of the spray orifice.

Thus, there remains a need for improved, superior sumps to address and solve the problem of medicament accumulation and/or deposition and the concomitant reduction in the dose available resulting in variations and inconsistencies of the actual dose administered, blockage of the spray orifice, and medicament flaking.

The inventors have devised a novel sump configuration, for use in an actuator for application with a pressurized aerosol container. In particular, the invention relates to the improvement and optimisation of drug delivery performance compared with conventional actuators incorporating angular sumps.

The improved sumps, according to the invention, may be incorporated in a wide range of aerosol actuators. The term "actuator" as used in the present invention includes all types of actuators presently available including but not limited to standard metered dose inhalers or breath operated inhalers. Breath-actuated devices are also known, and have been the subject of many patent applications. Thus, for example, GB 1288971; GB 1297993; GB 1335378; GB 1383761; GB 1392192; GB 1413285; W085/01880; GB 2204799; U.S. Pat. No. 4,803,978 and EP 0186280A describe inhalation-actuated dispensing devices for use with a pressurized aerosol dispensing container.

US 5,213,266 discloses an aerosol nozzle comprising a socket provided with an abutment surface for a hollow stem in an aerosol container and therein below an expansion chamber having a nozzle orifice in its wall. This document describes the importance of the precise location of the nozzle orifice in the wall of the expansion chamber.

Solvent-containing formulations are disclosed, for example, in WO 9947195 and WO 0037336. WO 9947195 discloses a valve for an aerosol container suitable for use in dispensing a quantity of the contents thereof. The valve components comprise a fluorinated polymer and/or a fluorinated coating which have been found to reduce drug deposition in the valve. HFA formulations are described which are preferably excipient free, but may contain solvents. WO 0037336 discloses a package for storing a pressurised container which is filled with a drug formulation at a predetermined pressure. The drug formulation includes a mixture of a drug and a propellant, and optionally a co-solvent such as ethanol.

A typical breath activated or operated device includes a dispensing container comprising a valve capable of releasing a metered amount of the aerosol contents, when an internal spring operating the valve is compressed by a sufficient amount. The dispensing device often comprises a chamber having a mouthpiece, air inlets, actuating means for causing the actuation of the valve in the dispensing container, a latching means for releasably retaining said metering valve in a charged position, and an inhalation responsive means for releasing the latch, such that a metered amount of aerosol compound is discharged into the region of the mouthpiece. The overall objective is to give co-ordination of discharge of medicament from the aerosol container with inhalation of the patient, thus allowing the required or intended dose of medicament to reach the bronchial passages of the lungs, or other target sites in the pulmonary system reliably.

The present invention works in conjunction with the medicament container, generally in the form of a cylindrical canister which, when activated, engages the valve stem against the rounded sump and discharges a metered dose of formulation. The rounded sump allows for smooth delivery, of a greater percentage of the metered dose, of the formulation as delivered by the metering valve of the canister towards the spray orifice and reduces the amount of measured dose lost in the delivery process by deposition and/or accumulation in and around the angles and corners of current sumps.

According to the invention we provide a metered dose aerosol actuator and a pressurised medicament container provided with a dispensing metering valve having a valve stem, comprising a housing adapted to receive the container, a stem block, extending upwards from the base of the housing and adapted to engage the valve stem by means of a cavity provided in the stem block, the stem block being provided with a spray orifice adapted to be in flow connection with the valve stem and the cavity, a sump extending downwards from the cavity, relative to the spray orifice, wherein the sump has a smooth, rounded interior surface.

The improved, rounded sump provides reduced deposition of the medicament by the provision of a continuous flow path for the formulation to be directed towards the spray orifice.

The improved sump is dimensioned and configured to include smooth surfaces which reduce the deposition of medicament accumulation and therefore improves product delivery through the spray channel to the spray orifice.

The actuator according to the invention, including the improved sump is used for formulations including ingredients such as excipients, surfactants or solvents, such as ethanol, glycerols, glycols and generally polyols, which are likely to promote deposition and/or accumulation.

The improved sump can be incorporated equally well in a wide range of inhalers such as a breath operated inhaler (BOI) and a metered dose inhaler (MDI).

With respect to current inhaler devices, the smooth, rounded sump is moulded in a continuous form to reduce the deposition of formulation around the sump area and improve the actuator performance, with the advantage that it contains no angles or corners where the medicament being delivered could accumulate and/or deposit, giving rise to a reduction in the dose available and blockage of the spray orifice

The smooth, rounded sump gives rise to an increase in the actual metered dose available at the spray orifice, via the spray channel, as a result of the occurrence for the blockage of said spray orifice.

The actuator of the invention, with the improved sump is particularly useful for the administration of medicinal pressurized aerosols, specifically those containing HFA propellants and more particularly those containing suspension formulations. The use of the improved sump can be considered for any actuator in which it would improve and/or optimise the actuator performance under laboratory conditions and under the conditions of or mimicking real life usage.

See WO 9947195 and WO 0037336 mentioned hereinabove for further discussion of the formulations.

Preferably, the sump is U-shaped.

The actuator may be manufactured from conventional materials used for actuators (i.e., from a plastics material, such as polypropylene, acetal or moulded polystyrene). It may however be manufactured from metal or another suitable material.

The invention many be embodies in other specific forms without departing from the subject matter of the claims. The present embodiments are therefore to be considered in all respects illustrative and not restrictive.

A preferred embodiment of the an actuator according to the invention will now by described, by way of example, with reference to the accompanying drawings, in which:
FIG. 1 is a side view in partial section of an aerosol actuator according to the invention fitted with a pressurised medicament container;
FIG. 2 is am expanded view of the stem-block of the actuator shown in FIG 1, in horizontal section, omitting the valve stem;
FIG. 3 is a vertical section of the stem-block shown in FIG. 2, along the line I-I.

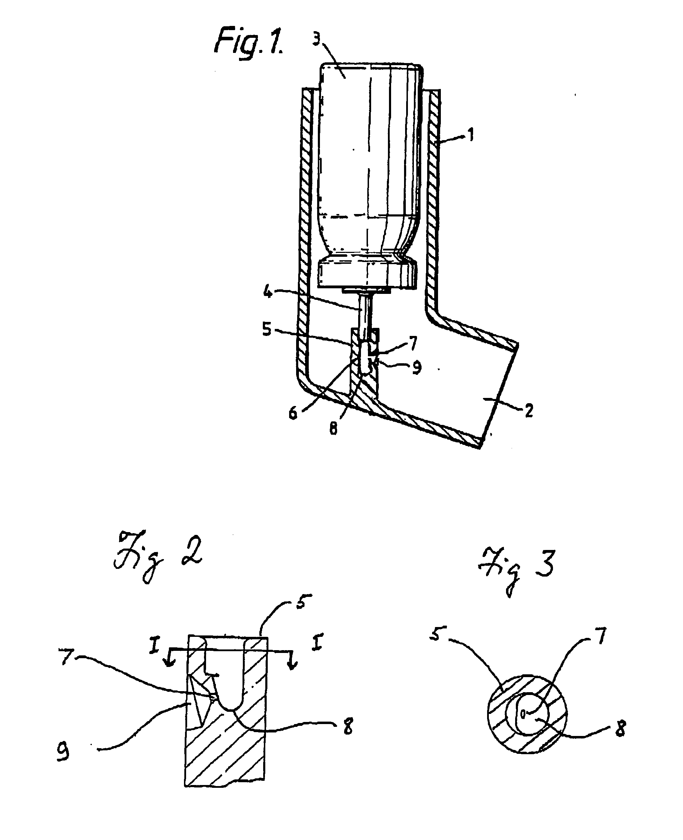

Referring first to FIG 1, an aerosol actuator comprises a generally cylindrical housing 1 having a mouthpiece 2. The housing 1 receives a container 3 of pressurised medicament, the container being provided at one end with a metering valve including a valve stem 4. The valve stem 4 is seated in the stem block 5. The stem block includes an internal cavity 6 provided with an outlet channel 7 which terminates on the external surface of the stem block 5, with a spray orifice 9, which is directed towards the mouthpiece 2. The valve stem 4, internal cavity 6 , outlet channel 7 and spray orifice 9 form a continuous flow path.:

As can be seen more clearly form FIG 2 and FIG 3, the internal cavity 6 tapers downwards to wards the base of housing 1, below the outlet channel 7, terminating in a smooth, rounded, "U" shaped sump, 8.

In use, a patient inhales at the mouthpiece 2 while simultaneously urging the medicament container 3 towards the stem block 5. The relative movement of the container 3 and the valve stem 4 causes the metering valve to discharge, dispensing a metered dose of pressurised formulation into the valve stem 4. The medicament then passes through the internal cavity 6 of the stem block, the outlet channel 7, and the spray orifice 8, after which medicament, in aerosol form is inhaled by the patient through the mouthpiece 2.

The sump 8 is moulded in a continuous form which gives the advantage that it contains no angles or corners where the medicament being delivered could accumulate and/or deposit, giving rise to a reduction in the dose available and blockage of the spray orifice 9 and thus improving the quantity and quality of the metered dose delivered from the container 3..

Medicament is not accumulated or deposited in the sump 8 due to the smooth rounded effect of the sump 8 which acts with the continuous flow path defined by the valve stem 4, internal cavity 6, spray channel 7 and spray orifice 9 to prevent blockage of the spray channel 7 or spray orifice 9 and improves product delivery through the spray orifice 9.

## Claims

1. An inhaler comprising a metered dose aerosol actuator and a pressurised medicament container (3) wherein the actuator is provided with a dispensing metering valve having a valve stem (4), comprising a housing (1) adapted to receive the container (3), a stem block (5), extending upwards from the base of the housing (2) and adapted to engage the valve stem (4) by means of a cavity (6) provided in the stem block (5), the stem block being provided with a spray orifice (9) adapted to be in flow connection with the valve stem (4) and the cavity, a sump (8) extending downwards from the cavity (6), relative to the spray orifice (9), wherein the container (3) contains a medicinal pressurized aerosol including an HFA propellant and a solvent **characterised in that** the sump (8) has a smooth, rounded interior surface and that the sump is moulded in a continuous form such that it contains no angles or corners where the medicament being delivered could accumulate and/or deposit.

2. An inhaler according to Claim 1, wherein the sump (8) is "U" shaped.

3. An inhaler according to Claim 1, wherein the solvent is selected from ethanol, glycerols, glycols and polyols.

4. An inhaler according to Claim 1, wherein the medicinal pressurized aerosol is a suspension formulation.

## Patentansprüche

1. Ein Inhalator bestehend aus einem Dosier-Sprüher und einem unter Druck stehenden Arzneimittelbehälter (3), in dem der Sprüher mit einem Dosierventil mit Ventilschaft (4) sitzt, bestehend aus einem Gehäuse (1) mit Fassungsvermögen für den Behälter (3), einem Schaftblock (5). Der Schaftblock ragt aus dem Gehäuseunterteil (2) heraus und sichert den Ventilschaft (4) durch eine Aushöhlung (6) im Schaftblock (5). Der Schaftblock ist mit einer Sprühöffnung (9) versehen, die im Arzneimittelfluss mit dem Ventilschaft (4) verbunden ist, und einer Aushöhlung, einem Sammelbehälter (8), der von der Aushöhlung (6) nach unten reicht und im Verhältnis zur Sprühöffnung (9) steht. Der Behälter (3) enthält ein unter Druck stehendes Arzneimittelspray mit HFA-Treibmittel und ein Lösungsmittel. Der Sammelbehälter (8) hat eine glatte, gerundete Innenfläche und ist kontinuierlich so geformt, dass keine Ecken oder Winkel vorhanden sind, an denen sich das zugeführte Arzneimittel sammeln bzw. ablagern könnte.

2. Ein Inhalator wie in Anspruch 1, bei dem der Sammelbehälter (8) U-förmig ist.

3. Ein Inhalator wie in Anspruch 1, bei dem das Lösungsmittel unter Ethanol, Glyzerolen, Glykolen und Polyolen gewählt wird.

4. Ein Inhalator wie in Anspruch 1, bei dem es sich bei dem unter Druck stehenden Arzneimittelspray um eine Suspension handelt.

## Revendications

1. Inhalateur avec un actionneur de type aérosol-doseur et un récipient sous pression contenant un produit pharmaceutique (3) où l'actionneur est fourni avec une valve de dosage équipée d'une tige de valve (4), et composé d'une enveloppe extérieure (1) adaptée pour contenir le récipient (3), d'un porte-tige (5) allant vers le haut à partir de la base de l'enveloppe (2) adapté pour recevoir la tige de la valve (4) grâce à une cavité (6) dans ledit porte-tige (5); ce porte-tige est doté d'un orifice de vaporisation (9) adapté pour être connectée et assurer le flux dans l'alignement de la tige de la valve (4) et de la cavité, d'un fond (8) allant vers le bas à partir de la cavité (6), par rapport à l'orifice de vaporisation (9), où le récipient (3) contient un aérosol de produit pharmaceutique sous pression, y compris un gaz propulseur HFA et un solvant, et dont le fond (8) possède une surface interne lisse et arrondie, et est moulé d'une seule pièce pour ne former aucun angle ou coin où le produit pharmaceutique pourrait s'accumuler ou former un dépôt.

2. Inhalateur selon la revendication 1, **caractérisé par** un fond (8) en forme de « U ».

3. Inhalateur selon la revendication 1, **caractérisé par** un solvant sélectionné parmi de l'éthanol, du glycérol, du glycol, et du polyol.

4. Inhalateur selon la revendication 1, **caractérisé par** un aérosol de produit pharmaceutique sous pression avec une formulation en suspension.
